# EUROPEAN PATENT SPECIFICATION

(11) **EP 2 369 923 B1**
(45) Date of publication and mention of the grant of the patent: **22.06.2016**
(21) Application number: 09764888.5
(22) Date of filing: 20.11.2009
(51) Int. Cl.: A01N 33/12, A01N 25/28, A61K 9/127, A61K 47/40, A01P 1/00

(54) **METHODS FOR INHIBITING GRAM-POSITIVE BACTERIA USING NON-PHOSPHOLIPID LIPID VESICULES**
VERFAHREN ZUR HEMMUNG VON GRAM-POSITIV-BAKTERIEN UNTER VERWENDUNG VON NICHT-PHOSPHOLIPID-ENTHALTENDEN LIPID-VESIKELN
METHODE D'INHIBITION DES BACTERIES GRAM-POSITIVE UTILISANT DES VESICULES LIPIDIQUES QUI NE CONTIENNENT PAS DES PHOSPHOLIPIDES

(30) Priority: 26.11.2008 US 118374 P
(43) Date of publication of application: 05.10.2011
(73) Proprietor: Viroblock SA, 1228 Plan-les-Quates (CH)
(72) Inventor: PELET, Thierry, 1219 Le Lignon (CH); MATHEUX, Franck, 74800 Vozerier (FR)
(74) Representative: KATZAROV S.A.
(86) International application number: PCT/IB2009/055242
(87) International publication number: WO 2010/061330

(56) References cited:
- EP-A1- 0 352 282
- WO-A2-2007/135523
- CAMPANHA M T N ET AL: "Interactions between cationic liposomes and bacteria: the physical-chemistry of the bactericidal action" JOURNAL OF LIPID RESEARCH, BETHESDA, MD, US, vol. 40, 1 January 1999 (1999-01-01), pages 1495-1500, XP007911923 ISSN: 0022-2275
- DITIZIO V ET AL: "A liposomal hydrogel for the prevention of bacterial adhesion to catheters" BIOMATERIALS, ELSEVIER SCIENCE PUBLISHERS BV., BARKING, GB, vol. 19, no. 20, 1 October 1998 (1998-10-01), pages 1877-1884, XP004161461 ISSN: 0142-9612
- DATABASE EMBASE ELSEVIER SCIENCE PUBLISHERS, AMSTERDAM, NL; 2007, A. Danion et al.: "Antibacterial activity of contact lenses bearing surface-immobilized layers of insect liposomes loaded with levofloxacin" XP002570513 Database accession no. emb-2007471744

## Description

### FIELD OF THE INVENTION

The present invention relates generally to the field of antibacterials and to the treatment, inhibition and prevention of disorders caused by bacteria. More particularly, this invention concerns a composition comprising at least one non-phospholpid lipid vesicle for inhibiting gram-positive bacteria.

### BACKGROUND OF THE INVENTION

Antibacterial resistance is a global clinical and public health problem that has emerged with alarming rapidity in recent years and undoubtedly will increase in the near future. Resistance is a problem in the community as well as in health care settings, where transmission of bacteria is greatly amplified. Because multiple drug resistance is a growing problem, physicians are now confronted with infections for which there is no effective therapy. The morbidity, mortality, and financial costs of such infections pose an increasing burden for health care systems worldwide, but especially in countries with limited resources. Strategies to address these issues emphasize enhanced surveillance of drug resistance, increased monitoring and improved usage of antimicrobial drugs, professional and public education, development of new drugs, and assessment of alternative therapeutic modalities.

Gram-positive bacteria are those that are stained dark blue or violet by Gram staining. Gram positive bacteria are generally characterised by having as part of their cell wall structure peptidoglycan as well as polysaccharides and/or teichoic acids. The peptidoglycans which are sometimes also called murein are heteropolymers of glycan strands, which are cross-linked through short peptides. Generally, gram-positive bacteria are the endospore formers and typically (though certainly not always) are the exotoxin releasers.

Classically, six gram-positive organisms are typically pathogenic in humans. Two of these, Streptococcus and Staphylococcus, are cocci (round bacteria). The remaining organisms are bacilli (rod-shaped bacteria) and can be subdivided based on their ability to form spores. The non-spore formers are Corynebacterium and Listeria, while Bacillus and Clostridium produce spores. The spore-forming bacteria can again be divided based on their respiration: Bacillus is a facultative anaerobe, while Clostridium is an obligate anaerobe.

One of the key contributors to the increase in mortality and morbidity due to bacterial infections is the increasing prevalence of drug-resistant bacteria. Examples of the seriousness of antibiotic resistance are methicillin-resistant *staphylococci* (MRSA), and the emergence of strains of vancomycin-resistant *S. aureus* which have become resistant to virtually all currently used antibiotics.

The heavy use of vancomycin to treat MRSA infections has in turn contributed to the emergence of new strains of *enterococci*, the third most prevalent cause of bacterial infection in the U.S., which are resistant to vancomycin. *Enterococcus* causes as many as 15 percent of bacterial endocarditis cases; it is also the cause of meningitis, and infections in the urinary tract, stomach and intestines. Infections caused by these vancomycin-resistant *enterococci* (VRE) frequently do not respond to any current therapies, and in many cases prove fatal.

Liposomes can be used to transport drugs for the delivery of pharmaceutical or cosmetic compositions. For example, International Patent Application WO96/12472 (Chinoin Gyógyszer És Vegyészeti Termékek Gyára RT et al.) disclosed a liposomic composition containing, as active ingredient, (-)-N- alpha -dimethyl-N-(2-propynylphenylethylamine) (selegilin) and/or salt thereof. The disclosed composition contains 0.1-40% by weight of selegilin and/or a salt thereof, 2 to 40% by weight of lipids, preferably phospholipids, 0 to 10% by weight of cholesterol, 0 to 20% by weight of an alcohol, 0 to 25% by weight of a glycol, 0 to 3% by weight of an antioxidant, 0 to 3% by weight of a preserving agent, 0 to 2% by weight of a viscosity influencing agent, 0 to 50% by weight of cyclodextrin or a cyclodextrin derivative and 30 to 90% by weight of water. This application also provides the administration of said composition for the treatment of Alzheimer's disease, Parkinson's disease, depression, stroke, motion sickness or myelitis.

Both phospholipids or non-phospholipids can be used to make liposomes. Compared to non-phospholipid liposomes, phospholipid liposomes are more costly, less stable and are unlikely to be available in the quantities needed for prophylaxes. Moreover, methods for producing phospholipid liposomes generally requires the use of organic solvents that are a major source of cellular toxicity. Also, it is more difficult to make phospholipid liposomes having low cholesterol content.

It is also known from WO2005030170 (Université Pasteur et al.) a method for initiating the controlled rupture of the membrane of a biocompatible phospholipid liposome, often called a furtive liposome, thereby releasing the liposome content to the environment thereof. A releasing agent, preferably an α-cyclodextrin, is embodied in the form of a biocompatible molecule.

US patent 5,561,062 (Varanelli et al.) provides an *in vitro* method of inactivating enveloped viruses by using paucilamellar lipid vesicles, preferably having non-phospholipids, and preparations useful in accomplishing this inactivation. The method is based on the discovery that paucilamellar lipid vesicles, preferably having non-phospholipids as their primary structural material, can fuse with enveloped virus and that the nucleic acid of the virus denatures shortly after the fusion. Generally, the paucilamellar lipid vesicle is filled with either an oil solution or a water solution, both containing a nucleic acid degrading agent.

Another patent application, EP 1 304 103 A1 (D.F.H Wallach) provides lipid vesicles wherein all said lipids are non phospholipids, as well as their use as vehicles particularly in therapeutic applications such as prevention of AIDS. These non-phospholipid lipid vesicles comprise at least one external stabilized bilayer comprising amongst other things a bilayer-modulating lipid chosen from the cholesterol family, an intravesicular aqueous space and at least one intravesicular micro-emulsion particle surrounded by an internal lipid monolayer. Inactivation of the HIV virus is due to the fusion of the non-phospholipid lipid vesicle with the membrane of said virus. This fusogenic property is probably due to the presence of cholesterol in the modulating lipid bilayer and there is no exchange of lipids between said non-phospholipid lipid vesicle containing cholesterol and the membrane of the HIV virus. Fusion between the nPLV described above and the membrane of an enveloped virus is not appropriate for *in vivo* inactivating said enveloped virus since it needs a long time to take place.

PCT publication WO 2007/135523 provides a composition for inactivating an enveloped virus comprising non-phospholipid Lipid Vesicles (nPLV). The inactivating effect is thought to be due to extraction of cholesterol from enveloped viruses by the non-phospholipid Lipid Vesicles. The non-phospholipid Lipid Vesicles can comprise an agent that enhances the lipid exchange between said nPLV and the membrane of said enveloped virus and can be cholesterol free.

Campanha et al discloses the antibacterial activity of non-phospholipid liposomes containing dioctadecyldimethylammonium bromide (DODAB) as lipid on gram positive and negative bacteria strains. At the DODAB concentrations used, there is no selective inhibition of gram positive (S. *aureus*) versus Gram negative (P. *aeruginosa*) bacteria.

None of the above publications discusses any selective effect of liposomes on gram-positive bacteria. Enveloped viruses and gram-positive bacterium are structurally different. Gram-positive bacterium have a cell wall composed of peptidoglycan (repeating units of N-acetylglucosamine and N-acetylmuramic acid) while enveloped viruses are enclosed within a lipoprotein membrane or envelope. It has been found that non-phospholipid lipid vesicles can inhibit growth or propagation of gram-positive bacterium. This is surprising given that the effect of non-phospholipid lipid vesicles on viruses was mediated by membrane lipid exchange between non-phospholipid lipid vesicles and viral envelope, while gram-positive bacterium have a cell wall composed of peptidoglycan.

There remains a need for a new method of inhibiting gram-positive bacteria that is rapid and efficient, *in vitro* as well *as in vivo.*

### SUMMARY OF THE INVENTION

The present invention is directed to a composition comprising at least one non-phospholipid lipid vesicle (nPLV) for use in the treatment, inhibition or prevention of a disorder associated with gram-positive bacteria wherein said nPLV comprises a lipid comprising a quaternary ammonium head group and the molar ratio of said lipid is from 0.01% to 1%.

In specific embodiments, the lipid is selected from the group consisting of: hexadecyl trimethylammonium bromide (HTAB), dimethyldihexadecyl ammonium bromide (DHAB), Cetrimonium bromide, cetyl trimethylammonium bromide (CTAB), hexadecyl trimethylammonium bromide, Cetrimonium chloride, cocamidopropyl betaine (CAPB), and tetra-n-butylammonium bromide (TBAB).

In general, the lipid is present in a molar ratio of from 0.01% to 1%. In one embodiment, the molar ratio is from 0.1% to 0.5%. In a specific embodiment, the molar ratio of said lipid is 0.3%.

The nPLV can be unilamellar, paucillamellar or multilamellar. In certain embodiments, the nPLV is cholesterol free. In other embodiments, the composition comprising the nPLV comprises a cyclodextrin or derivatives thereof. Preferably, the cyclodextrin is selected from the group consisting of: α-cyclodextrin, β-cyclodextrin, γ-cyclodextrin and a combination thereof. Suitable derivatives are selected from the group consisting of: dimethyl- β-cyclodextrin, trimethyl- β-cyclodextrin, randomly methylated- β-cyclodextrin, hydroxyethyl- β-cyclodextrin, 2-hydroxypropyl- β-cyclodextrin, 3-hydroxypropyl- β-cyclodextrin, 2,3-dihydroxypropyl- β-cyclodextrin, 2-hydroxyisobutyl- β-cyclodextrin, sulphobutylether- β-cyclodextrin, glucosyl- β-cyclodextrin, maltosyl- β-cyclodextrin and combinations thereof.

The present invention also provides nPLV compositions for use in the treatment, inhibition or prevention of a disorder associated with gram-positive bacteria. Preferably, the gram-positive bacteria is selected from the group consisting of: *Staphylococcus aureus*, *Staphylococcus epidermidis*, *Staphylococcus sp.* (Coagulase-negative), *Streptococcus pneumoniae* (Viridans group), *Streptococcus agalactiae* (group B), *Streptococcus pyogenes*, *Enterococcus sp., Bacillus anthracis*, *Bacillus oereus*, *Bifidobacteriu bifidum*, *Lactobacillus sp., Listeria monocytogenes, Nocardia sp.*, *Rhodococcus equi* (coccobacillus), *Erysipelothrix rhusiopathiae*, *Corynebacterium diptheriae, Propionibacterium acnes,* Actinomyces sp., *Clostridium botulinum, Clostridium difficile, Clostridium perfringens, Clostridium tetani, Mobiluncus sp.*, and *Peptostreptococcus sp.*

The present invention is also directed to a method for increasing the antibacterial activity of an object or objects comprising contacting said object or objects with a composition comprising at least one non-phospholipid lipid vesicle, wherein said nPLV comprises a lipid comprising a quaternary ammonium head group and the molar ratio of said lipid is from 0.01 % to 1%. In one embodiment, the nPLV comprises a second lipid comprising a quaternary ammonium head group. In another embodiment, the object to be disinfected is selected from the group consisting of: medical equipment, medical devices, and hospital supplies. In specific embodiments, the object is selected from the group consisting of a surface, surgical gloves, surgical instruments and personal masks. The composition of the invention can be in the form of an aqueous suspension or dispersion in hydrogels.

The present invention is also directed to a composition for use in the treatment, inhibition or prevention of a disorder associated with gram-positive bacteria in a subject comprising administering to said subject a therapeutically effective amount of a pharmaceutical composition comprising at least one non-phospholipid lipid vesicle, wherein said nPLV comprises a lipid comprising a quaternary ammonium head group and the molar ratio of said lipid is from 0.01 % to 1%, optionally in combination with one or more pharmaceutically acceptable carriers, wherein said pharmaceutical composition is delivered to a location proximate to said gram-positive bacteria. In one embodiment, the subject is a mammal, such as a human. In another embodiment, the disorder is an infection, such as a drug resistant and/or multiple drug resistant bacterial infection, e.g., one or more of methicillin resistant Staphylococcus aureus (MRSA), methicillin resistant Staphylococcus epidermidis (MRSE), methicillin resistant coagulase negative Staphylococci (MRCNS), vancomycin resistant Enterococcus faecalis, and/or vancomycin resistant Enterococcus faecium. In a particular embodiment, the infection is from gram positive bacteria.

The present invention is also directed to a composition for use in the treatment, inhibition or prevention of a disorder associated with gram-positive bacteria, comprising at least one non-phospholipid Lipid Vesicle (nPLV), wherein said nPLV comprises a lipid comprising a quaternary ammonium head group and the molar ratio of said lipid is from 0.01% to 1% and at least one other antibacterial agent. Suitable antibacterial agents include aminoglycosides, β-lactam antibiotics, cephalosporius, macrolides, miscellaneous antibiotics, penicillins, tetracyclines, antifungals, antimalarial agents, antituberculosis agents, antibacterials, leprostatics, miscellaneous anti-infectives, quinolones, sulfonamides, urinary anti-infectives, nasal antibiotics, opthalmic antibiotics, opthalmic antibacterials, opthalmicquinalones, opthalmic sulfonamides, skin and mucous membrane antibiotics, skin and mucous membrane antifungals, skin and mucous membrane antibacterials, skin and mucous membrane miscellaneous anti-infectives, skin and mucous membranescabicides and pedulicides, skin and mucous membrane antineoplasts, nitrofurans and oxazolidinones. The nPLV can be administered simultaneously or sequentially with said at least one other antibacterial agent.

The present invention is further directed to a kit for treating, inhibiting or preventing an infection of gram-positive bacteria in a subject, said kit comprising at least one non-phospholipid Lipid Vesicle (nPLV) in combination with reagents and/or instructions for use.

### BRIEF DESCRIPTION OF THE FIGURES

**Figure 1** shows the inhibitory effect of compositions comprising nPLVs on various gram-positive and gram-negative bacterium. PCE: polyoxyethylene cetyl ether, PA: palmitic acid, HTAB: hexadecyl trimethylammonium bromide, DHAB: dimethyldihexadecyl ammonium bromide. Compositions comprising nPLVs inhibit gram-positive bacteria.
**Figure 2** shows the minimum inhibitory concentration (MIC) for nPLV03 (99.9% PCE and 0.1% dimethyldihexadecyl ammonium bromide (DHAB)).

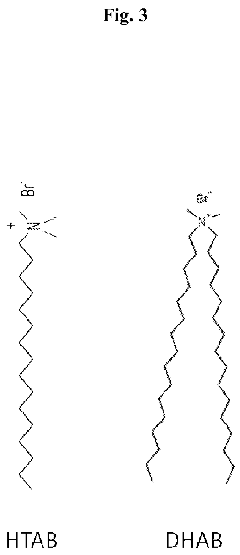

**Figure 3** shows a schematic view of the quaternary ammonium molecules used in the composition of nPLV03 and nPLV04.

### DETAILED DESCRIPTION OF THE INVENTION

"A" or "an" means "at least one" or "one or more."

As used herein, the terms "liposome" and "lipid vesicle" are used interchangeably to designate a small sphere made of lipid shells enclosing a central cavity mostly composed of an aqueous volume. The lipids are in the form of bimolecular layers, or lamellae, in an onion-like structure.

Non-phospholipid Lipid Vesicle (nPLV) refers to vesicles that are spherical structures made of material having high lipid content. These lipids are preferably comprised of non-phospholipids that can be selected from the group comprising the compounds set forth below in Table 2.

The terms "unilamellar", "paucilamellar", "multilamellar", as used herein, refer to the number of peripheral bilayers surrounding the central cavity of the liposome, in particular the nPLV of the invention. A unilamellar nPLV consists of one peripheral bilayer surrounding the central cavity whereas a multilamellar nPLV consists of more than 2 peripheral bilayers. Paucilamellar nPLV, which can be considered as a sub-class of the multilamellar nPLV, consists of 2 to 8 peripheral bilayers.

The molecular bilayers of nPLVs have a physical structure similar to classical phospholipid bilayers. For example, it has been shown that X-ray diffraction of C₁₆(PEG)₂ ether vesicles showed a simple and principal reflection, representing the thickness of a hydrated, double layer (5,8-6,1 nanometers) of amphiphile, with smaller spacing at higher cholesterol levels - fully analogous to phospholipid bilayers. The spacing of 6.1 nanometers corresponds to the maximum extension of two amphiphile molecules plus a layer of bound water (Mitchell, et al. 1983; Adam et al. 1984). Lantzsch et al. (1996) used fluorescent transfer techniques to determine the surfaces of surfactant type C₁₂(PEG)*_{N}* in 1-palmitoyl-2-oleoyl phosphatidylcholine /C₁₂(PEG)₁₋₈ liposomes. For *N* = 1-3, the expansion of surface is equivalent to a liquid-crystalline hydrocarbon phase per molecule of C₁₂(PEG)ₙ. For *N* = 4-8, the surface area per molecule of surfactant increased gradually, suggesting a rolled up configuration of the incorporated molecules, with two water molecules per ethylene glycol segment. Further, aqueous dispersions of 1,2-tetradecyl or 1,2-hexadecyl phosphatidylcholine accept large proportions of C₁₆(PEG)₄ (Mädler et al., 1998).

As used herein, the terms "to interact" and "interacting" are meant as having an effect one on another either by direct contact or at a distance.

Surprisingly, the Applicants have found that compositions comprising non-phospholipid lipid vesicles can inactivate gram-positive bacteria. This is surprising given that the effect of non-phospholipid lipid vesicles on viruses was mediated by membrane lipid exchange between non-phospholipid lipid vesicles and viral envelope, while gram-positive bacterium have a cell wall composed of peptidoglycan. Examples of gram-positive bacteria species include, but are not limited to, *Micrococcus, Peptococcus, Peptostreptococcus, Enterococcus, Bacillus, Clostridium, Lactobacillus, Listeria, Erysipelothrix, Propionibacterium, Eubacterium,* and *Corynebacterium.*

Gram Positive cocci include, for example, *Streptococcus* (e.g. *Streptococcus pneumoniae*), *Staphylococcus* (e.g. *Staphylococcus aureus*), and *Enterococcus.* Gram Positive Rods include, for example, *Corynebacteria* (i.e. *Corynebacterium diphtheriae*), *Listeria monocytogenes*, *Bacillus anthracis,* (i.e. *anthrax*). Gram Positive Branching Organisms include, for example, *Actinomycetes.* Gram positive bacteria include: *Bacillus anthracis; Bacillus subtilis; Clostridium botulinum; Clostridium perfringens; Clostridium tetani; Corynebacterium diphtheriae; Lactobacillus sup.; Listeria monocytogenes; Mycobacterium leprae; Mycobacterium tuberculosis; Mycoplasma pneumoniae; Staphylococcus aureus; Streptococcus spp.* Mycobacterium stain acid-fast but phylogentically are more closely related to the gram-positives than they are to the gram-negatives. Mycoplasma stain gram-negative but phylogentically are more closely related to the gram-positives than they are to the gram-negatives (it is their lack of a cell wall which leads to this confusing state). Additional gram positive bacteria are listed, for example, in volume 2 of Bergey's Manual, which contains six sections covering all gram-positive bacteria except the actinomycetes. Bacteria are distributed among these sections on the basis of their shape, the ability to form endospores, acid fastness, oxygen relationships, the ability to temporarily form mycelia, and other properties.

Exemplary gram-positive bacteria relevant in human medicine also include those listed in Table 1 below.

**Table 1**

| **Aerobic, Gram-positive cocci** | **Aerobic, Gram-positive rods** | **Anaerobic, Gram-positive rods** | **Anaerobic, Gram-positive cocci** |
|---|---|---|---|
| *Staphylococcus aureus* | *Bacillus anthracis* | *Actinomyces sp.* | *Peptostreptococcus sp.* |
| *Staphylococcus epidermidis* | *Bacillus oereus* | *Clostridium botulinum* | |
| *Staphylococcus sp. (Coagulase-negative)* | *Bifidobacteriu bifidum* | *Clostridium difficile* | |
| *Streptococcus pneumoniae (Viridans group)* | *Lactobacillus sp.* | *Clostridium perfringens* | |
| *Streptococcus agalactiae (group B)* | *Listeria monocytogenes* | *Clostridium tetani* | |
| *Streptococcu spyogenes* | *Nocardia sp.* | *Mobiluncus sp.* | |
| *Enterococcus sp.* | *Rhodococcus equi (coccobacillus)* | | |
| | *Erysipelothrix rhusiopathiae* | | |
| | *Corynebacterium diptheriae* | | |
| | *Propionibacterium acnes* | | |

A variety of Gram-positive organisms are capable of causing sepsis. The most common organisms involved in sepsis are *Staphylococcus aureus, Streptoccocus pneumoniae,* coagulase-negative *staphylococci, beta-hemolytic streptococci,* and *enterococci*, but any Gram-positive organism maybe involved (see, e.g., Bone, (1993) J. Critical Care 8:51-59). Thus, it is contemplated that the subject compositions and methods can be used as part of a therapeutic treatment or prevention program for sepsis involving Gram-positive bacteria.

It is further contemplated that the subject compositions and methods can be used as part of a therapeutic treatment or prevention program for bacteria infections caused by drug-resistant bacteria. Examples of such drug-resistant bacteria include methicillin-resistant *staphylococci* (MRSA), methicillin resistant *Staphylococcus epidermidis* (MRSE), methicillin resistant *coagulase negative Staphylococci* (MRCNS), vancomycin resistant *Enterococcus faecalis,* vancomycin resistant *Enterococcus faecium*, and vancomycin-resistant *S. aureus* which have become resistant to virtually all currently used antibiotics.

Non-phospholipid Lipid Vesicle (nPLV) disclosed herein preferably comprised of non-phospholipids that can be selected from the group comprising the compounds set forth below in Table 2.

**Table 2**

| **Classification** | **Hydrocarbon Chain** | **Bond** | **Head Group** |
|---|---|---|---|
| I. Fatty alcohol | C₁₂-C₂₀(0-1 unsaturation) | | -OH |
| 2. Fatty acid | C₁₂-C₂₀(0-1 unsaturation) | | -COOH |
| 3. Ethoxylated fatty alcohol | C₁₂-C₂₀(0-1 unsaturation) | -O | -(CH₂CH₂O)₂₋₅H |
| 4. Glycol ester of fatty acid | C₁₂-C₂₀(0-1 unsaturation) | -CO-O | -CH₂CH₂OH |
| 5. Ethoxylated fatty acid | C₁₂-C₂₀(0-1 unsaturation) | -CO-O | -(CH₂CN₂O)₂₋₁H |
| 6. Glycerol fatty acid monoester | C₁₂-C₂₀(0-1 unsaturation) | -CO-O | -CH₂CHOHCH₂OH |
| 7. Glycerol fatty | C₁₂-C₁₈(1 unsaturation) | -CO-O | -CH-CHO |
| acid diester | C₁₂-C₁₈(1 unsaturation) | -CO-O | -CH₂ |
| 8. Ethoxylated glycerol fatty acid ester | C₁₂-C₁₈(0 unsaturation) | -CO-O | -CH₂CHOHCH₂O(CH₂CH₂O)₉H |
| 9. Fatty acid diethanolamide | C₁₂-C₂₀(0-2 unsaturations) | -CO-N | -(CH₂CH₂OH)₂ |
| 10. Fatty acid dimethyl amide | C₁₂-C₂₀(0-2 unsaturations) | -CO-N | -(CH₃)₂ |
| 11. Fatty acid sarcosinates | C₁₂-C₁₈ (0 unsaturation) | 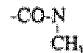 | -CH₂-COOH |
| 12. "Alkyd" | C₁₀(0 unsaturation) | O-CO | 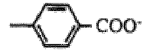 |
| 13. "Alkyd" | C₁₂-C₁₈(0-4 unsaturations) | -CO-O | -CH₂ |
| | C₁₂-C₁₂(0-4 unsaturations) | -CO-O | 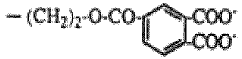 |

More preferably, the non-phospholipids of the invention are selected from the group consisting of polyoxyethylene cetyl ether (PCE), palmitic acid (PA), hexadecyl trimethylammonium bromide (HTAB), dimethyldihexadecyl ammonium bromide (DHAB) and oleic acid (OA), either alone or in combination.

In the present invention, the nPLV comprises a lipid comprising a quaternary ammonium head group. Such a quaternary ammonium containing lipid can be represented by the structure of NR₄⁺B⁻, wherein NR₄⁺ represents a quaternary ammonium cation, B⁻ represents an anion, and R can be same or different alkyl groups. Quaternary ammonium lipids are salts of quaternary ammonium cations with an anion. Quaternary ammonium cations can be synthesized by methods known in the art, for example, by complete alkylation of ammonia or other amines. Many quaternary ammonium lipids can be obtained commercially.

Exemplary quaternary ammonium lipids include hexadecyl trimethylammonium bromide (HTAB), dimethyldihexadecyl ammonium bromide (DHAB), Cetrimonium bromide, cetyl trimethylammonium bromide (CTAB), hexadecyltrimethylammonium bromide, Cetrimonium chloride, cocamidopropyl betaine (CAPB), tetra-n-butylammonium bromide (TBAB). In one embodiment, the quaternary ammonium lipid includes hexadecyl trimethylammonium bromide (HTAB) and dimethyldihexadecyl ammonium bromide (DHAB).

The ratio (e.g., molar ratio) of the non-quaternary ammonium lipid in the non-phospholipid lipid vesicle ranges from 0.01% to 1%, preferably from 0.1 % to 0.5%, more preferably from 0.1% to 0.3%. In one embodiment, the ratio is 0.3%.

In another embodiment, the nPLVs can be cholesterol-free (or substantially free of cholesterol), *i.e.* it does not comprise cholesterol (or, respectively, only traces of cholesterol), cholesterol derivatives such as for example PEG cholesterol, ionogenic cholesterol and surface stabilizing cholesterol, beta -sitosterol, ergosterol and phytosterol.

Usually, the nPLV of the invention is comprised in a composition.

In one embodiment, the composition comprising the nPLV can comprise an additional agent. In a particular embodiment, this agent is added to the composition and can be a cyclodextrin or a derivative thereof.

Cyclodextrins (CDs) are cyclic oligomers of glucose that can form water-soluble inclusion complexes with small molecules and portions of large compounds. Chemically they are cyclic oligosaccharides containing at least 6 D-(+) glucopyranose units attached by α - (1,4) glucosidic bonds. There are 3 natural CDs, α-, β-, and γ-CDs, which differ in their ring size and solubility. These biocompatible, cyclic oligosaccharides do not elicit immune responses and have low toxicities in animals and humans. Cyclodextrins are used in pharmaceutical applications for numerous purposes, including improving the bioavailability of drugs. β -CD can selectively extract cholesterol from cellular membranes. High concentrations of β -CD show cellular toxicity and can induce either cell lysis or cellular cell death (apoptosis).

Derivatives of CD are disclosed in U.S. Patent Number 5,760,017 (inventors: Djedaini-Pilard et al.) and International Application WO91/13100 (inventors: Coates et al.), the disclosure of which is also incorporated herein by reference. Examples of CD derivatives comprise, but are not limited to dimethyl- β-cyclodextrin, trimethyl- β-cyclodextrin, randomly methylated- β-cyclodextrin, hydroxyethyl- β-cyclodextrin, 2-hydroxypropyl- β-cyclodextrin, 3-hydroxypropyl- β-cyclodextrin, 2,3-dihydroxypropyl- β-cyclodextrin, 2-hydroxyisobutyl- β-cyclodextrin, sulphobutylether- β-cyclodextrin, glucosyl- β-cyclodextrin and maltosyl- β-cyclodextrin.

Usually, the agent is added to the composition. To this end a suitable concentration of CD is prepared in water or PBS and added to the composition so as to obtain the required concentration.

Preferably, the concentration of cyclodextrin or cyclodextrin derivatives in the composition of the invention is between 0.01 mM and 50 mM. Most preferably, this concentration is between 0.1 mM and 10 mM. At such a low concentration, β -CD has limited effect on cellular integrity.

Methods of manufacturing nPLVs, and the nPLVs of the invention, are also described in more detail in Example 1 or in U.S. Pat. No. 4,911,928, U.S. Pat. No. 5,147,723, U.S. Pat. No. 5,032,457, U.S. Pat. No. 4,895,452, U.S. patent application Ser. No. 761,253, and WO 2007/135523.

Also encompassed by the present invention is a pharmaceutical composition characterized in that it comprises a pharmaceutically acceptable amount of the nPLVs disclosed herein, optionally in combination with one or more pharmaceutically acceptable carriers.

In a further embodiment, the pharmaceutical composition further comprises an enhancer agent as described above. Preferably, the enhancer agent is at least one cyclodextrin or derivatives thereof.

The phrase "pharmaceutically acceptable" refers to molecular entities and compositions that are physiologically tolerable and do not typically produce an allergic or similar untoward reaction, such as gastric upset, dizziness and the like, when administered to a human.

Acceptable carriers, excipients, or stabilizers are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; low molecular weight (less than 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes.

The form of administration of the pharmaceutical composition may be systemic or topical. For example, administration of such a composition may be various parenteral routes such as subcutaneous, intravenous, intradermal, intramuscular, intraperitoneal, intranasal, transdermal, buccal routes, preferentially by inhalation, or via an implanted device, and may also be delivered by peristaltic means.

The pharmaceutical composition, as described herein, may also be incorporated or impregnated into a bioabsorbable matrix, with the matrix being administered in the form of a suspension of matrix, a gel or a solid support. In addition the matrix may be comprised of a biopolymer.

In case the formulations to be used for in vivo administration must be sterile, this is readily accomplished for example by using sterile compounds for the preparation of the composition of the invention.

It is understood that the suitable dosage of the pharmaceutical composition of the present invention will be dependent upon the age, sex, health, and weight of the recipient, kind of concurrent treatment, if any and the nature of the effect desired.

The appropriate dosage form will depend on the disease, the nPLV, the enhancer agent and the mode of administration; possibilities include a spray or other aerosol means of delivery to the respiratory passages. Other ways of topically applying the inhibiting solution include creams, mouthwashes, dental pastes, eye drops, solutions, ointments, gels such as vaginal gels, and lubricants such as condom lubricants.

An optional dose of the compound for treatment of a bacterial (such as a gram positive bacteria) infection is 1 to 50 mg/kg, or 1 to 20 mg/kg, of body weight per day, more generally 0.1 to 100 mg per kilogram body weight of the recipient per day. The effective dosage range of the pharmaceutically acceptable salts and prodrugs can be calculated based on the weight of the parent nucleoside to be delivered. If the salt or prodrug exhibits activity in itself, the effective dosage can be estimated as above using the weight of the salt or prodrug, or by other means known to those skilled in the art.

Optionally, the active ingredient should be administered to achieve peak plasma concentrations of the active compound of from 0.2 to 70 M, e.g., 1.0 to 10 uM. This maybe achieved, for example, by the intravenous injection of a 0.1 to 5% solution of the active ingredient, optionally in saline, or administered as a bolus of the active ingredient. The concentration of active compound in the drug composition will depend on absorption, inactivation and excretion rates of the drug as well as other factors known to those of skill in the art. It is to be further understood that for any particular subject, specific dosage regimens should be adjusted according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

The compound is conveniently administered in unit any suitable dosage form, including but not limited to one containing 7 to 3000 mg, or 70 to 1400 mg of active ingredient per unit dosage form. A dosage of 50-1000 mg is optional.

The present invention also provides a composition for use in the prevention, inhibition or treatment of a bacterial infection, in a host, for example an animal, and typically a human, comprising administering a therapeutic amount of a nPLV composition of the present invention, optionally in a pharmaceutically acceptable carrier or diluent where the bacterial infection is due to a gram-positive bacteria.
The present disclosure also provides a method for inhibiting gram-positive bacteria comprising the step of contacting said gram-positive bacteria with a composition comprising one or more nPLVs, wherein said nPLV comprises a lipid comprising a quaternary ammonium head group and the molar ratio of said lipid is from 0.01% to 1%. Inhibition of bacterial replication or treatment of an infection in a cell can be measured by showing a reduction in bacterial replication in a cell to a level lower than the level in an otherwise identical cell, which was not administered the compound or composition of the invention. The reduction can be by 80%, 85%, 90%, 95%, 99.9% or more. The level of bacterial replication in a cell can be assessed by any known methods. For example, the level of bacterial replication in a cell can be assessed by evaluating the number of bacterial particles or amount of a bacterial component, such as a bacterial protein, a bacterial enzyme, or bacterial nucleic acid, in the cell or in fluid or debris associated with the cell. The number of infectious bacteria in a cell can be evaluated, for example, in a plaque assay. The level of a bacterial component such as a bacterial protein or enzyme in a cell can be evaluated using standard analytical techniques of protein biochemistry, such as, for example, using an activity assay for a bacterial enzyme, or using Western blotting or quantitative gel electrophoresis for a bacterial protein. Bacterial nucleic acid levels in a cell can be evaluated using standard analytical techniques such as Northern blotting and Southern Blotting or quantitation by polymerase chain reaction (PCR). nPLVs that can be used in the methods disclosed herein include those that are disclosed above. The nPLVs used for inhibiting gram-positive bacteria comprise a quaternary ammonium head group. Such quaternary ammonium containing lipids are disclosed above. In another embodiment, the quaternary ammonium containing lipids contained in the nPLVs include hexadecyl trimethylammonium bromide (HTAB) and dimethyldihexadecyl ammonium bromide (DHAB). In a further embodiment, the nPLV can comprise an additional agent which is added to the composition. In a particular embodiment, this agent can be a cyclodextrin. Preferably, the agent is a cyclodextrin or a derivative thereof. Cyclodextrins or derivatives thereof are disclosed above. In still a further embodiment, the nPLVs can be cholesterol-free.

The gram-positive bacteria that can be inhibited by the nPLVs include those disclosed above, such as *Staphylococcus, Streptococcus, Micrococcus, Peptococcus*, *Peptostreptococcus, Enterococcus, Bacillus, Clostridium, Lactobacillus, Listeria, Erysipelothrix, Propionibacterium, Eubacterium, and Corynebacterium, Streptococcus* (e.g. *Streptococcus pneumoniae*), *Staphylococcus* (e.g. *Staphylococcus aureus*), and *Enterococcus.* Gram Positive Rods include, for example, *Corynebacteria* (i.e. *Corynebacterium diphtheriae*), *Listeria monocytogenes, Bacillus anthracis* (i.e. *anthrax*) and *Erysipelothrix rhusiopathiae.* Gram Positive Branching Organisms include, for example, *Actinomycetes.* Gram positive bacteria include: *Bacillus anthracis; Bacillus subtilis; Clostridium botulinum; Clostridium perfringens; Clostridium tetani; Corynebacterium diphtheriae; Lactobacillus spp.; Listeria monocytogenes; Mycobacterium leprae; Mycobacterium tuberculosis; Mycoplasma pneumoniae; Staphylococcus aureus; Streptococcus spp.* Mycobacterium stain acid-fast but phylogentically are more closely related to the gram-positives than they are to the gram-negatives. Mycoplasma stain gram-negative but phylogentically are more closely related to the gram-positives than they are to the gram-negatives (it is their lack of a cell wall which leads to this confusing state). Additional gram positive bacteria are listed, for example, in volume 2 of Bergey's Manual, which contains six sections covering all gram-positive bacteria except the actinomycetes. Bacteria are distributed among these sections on the basis of their shape, the ability to form endospores, acid fastness, oxygen relationships, the ability to temporarily form mycelia, and other properties. In one embodiment, the gram-positive bacteria that can be inactivated includes those relevant in human medicine (e.g., those listed in Table 1). In particular embodiment, the nPLVs can be used to inactivate drug-resistant bacteria including drug-resistant bacteria include methicillin-resistant *staphylococci* (MRSA), methicillin resistant *Staphylococcus epidermidis* (MRSE), methicillin resistant *coagulase negative Staphylococci* (MRCNS), vancomycin resistant *Enterococcus faecalis*, vancomycin resistant *Enterococcus faecium*, and vancomycin-resistant *S. aureus.*

Minimum inhibitory concentration (MIC) is the lowest concentration of an antimicrobial that will inhibit the visible growth of a microorganism after overnight incubation.

Generally, MICs can be determined by agar or broth (e.g., LB broth) dilution methods. A standard method of determining MIC for a composition comprising nPLVs includes: growing inoculums of bacteria (e.g., 5x10⁵ bacteria/ml) overnight in LB broth medium in presence of various concentration of a composition comprising nPLVs, each culture sample is then serially diluted and plated on an agar plate, the agar plate is then incubated overnight at 37 °C for titer determination. Disclosure of such a method can be found in "Determination of minimum inhibitory concentrations" by Jennifer M. Andrews in Journal of Antimicrobial Chemotherapy (2001) 48, Suppl. S1, 5-16. In addition, guidelines determination of MICs can be found from organizations such as the Clinical and Laboratory Standards Institute (CLSI), the British Society for Antimicrobial Chemistry (BSAC) or the European Society of Clinical Microbiology and Infectious Disease (EUCAST). There are also several commercial methods available, including the Etest strips and the Oxoid MICEvaluator method.

MICs are important in diagnostic laboratories to confirm resistance of microorganisms to an antimicrobial agent. Clinically, the minimum inhibitory concentrations can be used to determine the amount of antibiotic that a patient will receive, as well as the type of antibiotic used. Such determinations can lower the opportunity for microbial resistance to specific antimicrobial agents.

MICs for nPLVs against various gram-positive bacteria can be determined using standard methods described above or other methods known in the art. These MICs can be used to determine the minimum amount of the respective nPLV that can be used for inhibiting gram-positive bacteria.

The present disclosure further provides a composition for use in the treatment or prevention of a disease associated with gram-positive bacteria in a subject comprising the step of administering to said subject the pharmaceutical composition as described herein, to a location proximate to said gram-positive bacteria. Possible modes of delivery include a spray or other aerosol means of delivery to the respiratory passages, creams, mouthwashes, dental pastes, solutions, ointments, gels such as vaginal gels, eyes drops and lubricants such as condom lubricants, creams, lotions, or solutions for external application to skin and mucosal surfaces, including the cornea, dermal cuts and abrasions, burns, and sites of bacterial infection.

Interaction of the composition of the invention with gram-positive bacteria in the airways blocks bacteria infectivity, thereby, propagation in the lungs (individual prophylaxis-treatment). Physiological processes flush out the composition and inactivated bacteria. Moreover, being partially or completely inactive, bacteria are limited in their spread in the surrounding population when expelled by coughing or sneezing (population prophylaxis).

The term "treatment" refers to therapeutic treatment, while the term "prevention" refers to prophylactic or preventative measures. Those (subjects) in need of treatment may include those already with a disorder as well as those in which a disorder is to be prevented. The subject to be treated herein may have been diagnosed as having a disorder or may be susceptible to a disorder.

Preferably, the subject is an animal or a human.

Most preferably the term animal refers to domestic and farm animals (e.g. poultries), and zoo, sports, or pet animals, such as dogs, horses, pigs, cats, cows, monkeys etc...

Embraced by the scope of the present invention is also the use of the pharmaceutical composition, as described herein, in the preparation of a medicament for the treatment or prevention of a gram-positive bacteria-associated disease.

Also encompassed in the present invention is the use of the composition of the invention in the preparation of a large-scale biocompatible disinfectant. Accordingly, the composition of the invention is diluted as needed to prepare simple aqueous suspensions, or formulated as dispersions in hydrogels, pastes or sprays.

In addition to the described active components, the disinfectant preparations of the invention may contain other typical components depending on the desired use of the formulation. In particular, an acidifier may be used to keep the pH range of the disinfection solution below 6. Suitable solvents for the protonated compounds and/or the other active ingredients may be employed, and preferably are water or water miscible organic solvents. Solutions such as these may be readily sprayed using compressed air or any other propellants known by those in the art.

These disinfectant preparations of the invention are suitable for surface disinfection in medically-related environments, such as hospitals, veterinary clinics, dental and medical offices and the like. Use of solutions of the invention in the sterilization of surgical instruments is especially preferred. These disinfectant preparations are also useful in public areas such as schools, public transport, restaurants, hotels and laundries. The disinfectants also find use in home as sanitizers for toilets, basins, and kitchen areas. The disinfectant preparations comprising the compositions disclosed herein can be used to disinfect medical devices, surgical instruments, surgical glows or personal masks. The disinfectants may also be useful in surgical settings, both for the medical staff and to sterilize the area of surgery on the patient. For example, surgical instruments can be coated with the compositions of the invention to provide for a sterile coating prior to surgery.

The present disclosure also provides the use of the composition of the invention, in the preparation of an antibacterial coating agent. This coating agent may then be used to cover, for example, surgical glows, surgical devices, surgical apparatus, male condoms and personal masks, or for coating indwelling catheters and similar implants which are susceptible to harboring bacterial infection.

Another aspect of the present invention is to provide a kit for inhibiting gram-positive bacteria, said kit comprising an nPLV composition of the invention, optionally with reagents and/or instructions for use.

It is possible that drug-resistant variants of bacteria can emerge after prolonged treatment with an anti-bacterial agent. The efficacy of a drug against the bacterial infection can be prolonged, augmented, or restored by administering the compound in combination or alternation with a second, and perhaps third, anti-bacterial agent, for example with a different site of activity than the principle drug. Alternatively, the pharmacokinetics, biodistribution or other parameter of the drug can be altered by such combination or alternation therapy. In general, combination therapy is typical because it induces multiple simultaneous stresses on the bacteria.

Accordingly, the kit of the present invention may further comprise a separate pharmaceutical dosage form comprising an additional anti-bacteria agent. Suitable antibiotic agents are disclosed, e.g. in Physician's Desk 30 Reference (PDR), Medical Economics Company (Montvale, N.J.), (53rd Ed.), 1999; Mayo Medical Center Formulary, Unabridged Version, Mayo Clinic (Rochester, Minn.), January 1998; Merck Index An Encyclopedia of Chemicals, Drugs and Biologicals, (11th Ed.), Merck & Co., Inc. (Rahway, N.J.), 1989; University of Wisconsin Antimicrobial Use Guide, http://www.medsch.wisc.edu/clinsci/ 5amcg/amcg.html; Introduction on the Use of the Antibiotics Guideline, of Specific Antibiotic Classes, Thomas Jefferson University, http://jeffiine.tju.edu/CWIS/OAC/antibiotics_guide/intro.html; and references cited therein.

Non-limiting examples of antibiotics classes include Sulfonamides, Penicillins, Cephalosporins, Aminoglycosides, Chloramphenicol, Tetracyclines, Macrolides, Lincosamides, Streptogramins, Glycopeptides, Rifamycins, Nitroimidiazoles, Quinolones, Trimethoprim, Oxazolidinones, Lipopeptides.

Specific compounds include, for example, Amikacin (amikacin sulfate); Craramyein (gentamicin sulfate); Nebcin (tobramycin sulfate); Netromycin (netilmicin sulfate); Streptomycin Sulfate; and TOBI (tobramycin), Azactam (aztreonam); Cefotan (cefotetan); Lorabid (loracarbef); Mefoxin (cefoxitin); Merrem (meropenem); and Primaxin (imipenem and cilastatin for injectable suspension); Ancef (cefazolin); Ceclor (cefaclor); Cedax (ceffibuten); Cefizox (ceffizoxime sodium); Cefobid (cefoperazone sodium); Ceftin (cefuroxime axetil); Cefzil (cefprozil); Ceptaz (ceftazidime); Claforan (cefotaxime); Duricef (cefadroxil monohydrate); Fortaz (ceftazidime); Keflex (cephalexin); Keftab (cephalexin HCl); Kefurox (cefuroxime); Kefzol (cefazolin); Mandol (cefamandole nafate); Maxipime (cefepime HCl); Monocid (cefonicidsodium); Omnicef (cefdinir); Rocephin (ceftriaxone); Suprax (cefixime); Tazicef (ceftazidime); Tazidime (ceftazidime); Vantin (cefpodoxime proxetil); and Zinacef5 (cefuroxime); Biaxin (clarithromycin); Dynabac (dirithromycin); E.E.S. 200 (Erythromycin Ethylsuccinate); E.E.S. 400 (Erythromycin Ethylsuccinate); EryPed 200 (Erythromycin Ethylsuccinate); EryPed 400 (Erythromycin Ethylsuccinate); Ery-Tab (Erythromycin delayed-release tablets); Erythrocin Stearate (Erythromycin stearate); Ilosone (erythromycinestolate); PCE Dispertab (erythromycin particles in tablets); Pediazole (erythromycin ethylsuccinate and sulfisoxazole acetyl for oral suspension); Tao (troleandomycin); Zithromax (azithromycin); and Erythromycin; Cleocin HCl (clindamycin hydrochloride); Cleotin Phosphate (elindamycin phosphate); Coly-Mycin M (colistimethate sodium); and Vancocin HCl (vancomycin hydrochloride); Amoxil (amoxicillin); Augmentin (amoxicillin/clavulanate potassium); Bicillin C-R 900/300 (Penicillin G benzathine and Penicillin G procaine suspension); Bicillin C-R (Penicillin G benzathine and Penicillin G procaine suspension); Bicillin L-A (Penicillin G benzathine suspension); Geoeillin (carbencillin indanyl sodium); Mezlin (sterile mezlocillinsodium); Omnipen (ampicillin); Pen-Vee K (penicillin V potassium); Pfizerpen (penicillin G potassium); Pipracil (piperacillin sodium); Speetrobid (bacampicillin-HCl); Ticar (tiearcillin disodium); Timentin (ticarcillin disodium and clavulanate potassium); Unasyn (ampicillin sodium/sulbactam sodium); Zosyn (piperacillin sodium and tazobactam sodium); and Dicloxacillin Sodium; Achromycin V (tetracycline HCl); Declomycin (demeclo-cycline HCl); Dynacin (minocylcine HCl); Minocin (minocycline hydrochloride); Monodox (Doxycycline monohydrate capsules); Terramycin (oxytetracyline); Vectrin (minocycline hydrochloride); Vibramycin Calcium (doxycycline sodium); Vibramycin Hyclate (doxycycline hyclate); Vibramycin Monohydrate (doxycycline monohydrate); Vibra-Tabs (doxycycline-hydrate); Declomycin (demeclocycline HCl); Vibramycin (doxycycline); Dynacin (Minocyline HCl); Terramycin (oxytetracycline HCl); Achromycin V capsules5 (tetracycline HCl); Linco-mycins; and Cleotin HCl (clindamycin HCl); Abelcet (amphotericin B lipid complex); AmBisome (amphotericin B); Amphotec (amphotericin B cholesterol sulfatecomplex); Ancobon (flucytosine); Diflucan (fluconazole); Fulvicin P/Gamma (ultramicrosize griseofulvin); Fulvicin P/G 165 and 330 (ultramicrosize griseofulvin); Grifulvin V (griseofulvin); Gals-PEG (gxiseofulvin ultramicrosize); Lamisil (terbinafine hydrochloride); Nizoral (ketoconazole); Amphotericin B; Lotrimin (clotrimazole); Dapsone tablets (dapsone); Diflucan (fluconazole); Monistat-Derm cream (miconazole); Mycostalin Crc am (nystatin); and Sporanox (itraconazole); Aralen hydrochloride (chloroquine HCl); Aralen phosphate (chloroquine phosphate); Dataprim (pyrimethamine); Ladam (mefloquine HCl); and Plaquenil (hydroxychloroqnine sulfate); Capastat sulfate (capreomycinsulfate); Myambutol (ethambutol hydrochloride); Mycobutin (rifabutin capsules); Nydrazid (isoniazid injection); Paser (aminosalicylic acid); Prifiin (rifapentine); Pyrazinamide tablets (pyrazinamide); Rifadin (rifampin capsules); Rifadin IV (rifampin for injection); Rifamate (rifampin and isoniazid); Rifater (rifampin, isoniazid and pyrazinamide); Seromycin (cycloserine capsules); Streptomycin-Sulfate; Tice BCG (3CG vaccine); Cycloserine (seromycin capsules); Urised (Methenamine); and Trecator-SC (ethionamide tablets); Alferon N (interferon alfa-n3); Crixivan (indinavir sulfate); Cytovene (ganciclovir); Cytovene-IV (ganciclovir sodium); Epivir (lamivudine); Famvir (famciclovir); Flumadine (rimantadine HCl); Foscavir (foscamet sodium); Hivid (zalcitabine); Intron A (interferon alfa-2b); Invirase (saquinavir mesylate); Norvir (ritonavir); Rebetron combination therapy, which contains Rebetrol (ribavirin) and Intron A (inteferon alfa-2b); Rescriptor (delavirdine mesylate); Retrovir (ziduvudine); Retrovir IV (ziduvudine); Symmetrel (amantadine HCl); Synagis (palivizumab); Valtrex (valacyclovir HCl); Videx (didanosine); Viracept (nelfinavir mesylate); Viramune (nevirapine); Virazole (ribavirin); Vistide (cidofovir); Zerit (stavudine (d4T)); Symmetrel Syrup (amantadine HCl); Combivir Tablets (lamiduvine); and Zovirax (acyclovir); Dapsone Tablets (dapsone); Daraprim (pyrimethamine); Flagyl 375 (metronidazole); Flagyl ER Tablets (metronidazole); Flagyl I.V. (metronidazole); Furoxone (furazolidone); Mepron (atovaquone); and Neutrexin (tfimetrexate glucuronate); Cipro (ciprofloxacin HCl); Floxin (ofloxacin); Levaquin (levofloxacin); Mazaquin (lomefioxacin HCl); Noroxin (norfloxacin); Penetrex (enoxacin); Raxar (grepafloxacin HCl); Trovan (trovafioxacin mesylate); and Zagam (sparfloxacin); Bactrim.(trimethoprim and sulfamethoxazole); Bactrim DS (Irimethoprim and sulfamethoxazole double strength); Pediazole (erythromycin ethylsuccinate and sulfisoxazole acetyl); Septra (trimethoprim and sulfamethoxazole); Septra DS (trimethoprim and sulfamethoxazole); Co-Trimoxazole, Sulfadiazine, Battrim I.V. Infusion (sulfamethoxazole); Sulfapyridine and Pediazole (erythromycin ethylsuccinate and sulfisoxazole acetyl); Furadantin (nitrofurantoin); Macrobid (nitrofurantoin monohydrate macrocrystals); Macrodantin (nitrofurantoin macrocrystals); Monurol Sachet (fosfomycin tromethamine); NegGram Caplets (nalidixic acid); Septra (trimethoprim and sulfamethoxazole); Septra DS (trimethoprim and sulfamethoxazole); Urised (a combination of the antisepticsmethenamine, methylene blue, phenyl salicylate, benzoic acid and parasympatholytics (atropine sulfate) hyoscyamine); (oxytetracycline HCl, sulfamethizole and phenazopyridine HCl); (methenamine mandelate); Bactroban (mupirocin); Chloromycetin opthalmic (chloramphenical); Cortisporin (neomycin and polymyxin [3 sulfates and hydrocortisone acetate cream); Ilotycin (erythromycin opthahnic ointment); NeoDecadron (neomycin sulfate--dexamethasone sodium phosphate); Polytrim (tfimethoprim and polythyxin [3 sulfate opthalmic solution); Terra-Cortril (oxytetracycline HCl and hydrocortisone acetate); Terramycin (oxytetracycline); and TobraDex (tobramycin and dexamethasone opthalmic suspension and ointment); Vita-A opthalmic ointment, (vidatabine); (norfloxacinopthalmic solution; Ciloxan opthalmic solution and ointment (Ciprofloxacin HCl); and Ocuflox opthalmic solution (ofioxacin), Blephamide opthalmicointment (sulfacetamide sodium and prednisolone acetate); and Blephamideopthalmic suspension (sulfacetamide sodium and predrdsolone acetate); A/T/S (erythromycin); Bactroban (mupirocin); Benzamycin (erythromycin-benzoyl peroxide topical gel); Betadine (povidone-odine); Cleotin T (clindamy cinphosphate topical solution); Clindets (clindamycin phosphate pledgets); Cortispofin (neomycin, polymyxin B sulfates and hydrocortisone acetate cream); Emgel (erythromycin); Erycette (erythromycin topical solution); Garamycin (gentamicin sulfate); Klaron (sodium sulfacetamide lotion); Mycostatin (nystatin cream); Theramycin Z (erythromycin topical solution); T-Stat (erythromycin); Chloromycetin (chloramphenicol opthalmic ointment); Cortisporin (neomycin and polymyxin B sulfates, bacitracin zinc and hydrocortisone opthalmic ointment); Ilotycin (erythromycin); NeoDeeadron (neomycin sulfate-dexamethasone sodium phosphate); Polytrim (trimethoprim and polymyxin B sulfate); Terra-Cortril (oxytetracycline HCl and hydrocortisone acetate); Terramycin (oxytetracycline); Exelderm (sulconazole nitrate); Fungizone (amphotericin B oral suspension); Lamisil (terbinafine hydrochloride cream); Loprox (ciclopiroxolamine); Lotrimin (clotrimazole); Lotrisone (clotrimazole and betamethasone diproprionate); Mentax (butenafine HCl); Monistat-Denn (miconazole nitrate); Mycelex (clotrimazole); Mycostatin (nystatin); Naffin (nattifine HCl); Nizoral Ocetoconazole); Nystop (nystatin); Oxistat (oxiconazole nitrate); Selsun R.sup.x (2.5% selenium sulfide lotion); and Spectazole (econazole nitrate); Denavir (penciclovir cream); and Zovirax (acyclovir); Benzashave Coenzoyl peroxide); Betadine (povidone-iodine); Betasept (chlorhexidine gluconate); Cetaphil (soap substitute); Clorpactin WCS-90 (sodium oxychlorosene); Dapsone Tablets (dapsone); Desquam-E Coenzoyl peroxide); Desquam-X (benzoyl peroxide); Hibiclens (chlorhexidine gluconate); Hibistat (ehlorhexidine gluconate); Impregon (tetrachlorosalicylanilide 2%); MetroCream (metronidazole); MetroGel (metronidazole); Noritate (metronidazole); pHisoHex (hexachlorophene detergent cleanser); Sulfacet-R (sodium sulfacetamide 10% and sulfur 5%); Sulfamylon (materfide acetate); Tfiaz Coenzoyl peroxide); and Vanoxide-HC Coenzoyl peroxide hydrocortisone); Acticin (permethrin); Elimite (permethrin); Eurax (crotamiton); Efudex (fluoro-uracil); Fluoroplex, rifapentine, quinupristin/dalfopristin, moxifloxacin, gatifloxacin, linezolid, cefditoren pivoxil, ertapenem, gemifloxacin, daptomycin, telithromycin, and combinations thereof.

Alternatively, the nPLV composition of the invention may further comprise an additional anti-bacteria agent listed above and combinations thereof.

In combination therapy, effective dosages of two or more agents are administered together, whereas in alternation or sequential-step therapy, an effective dosage of each agent is administered serially or sequentially. The dosages given will depend on absorption, inactivation and excretion rates of the drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions. In some embodiments, an anti-bacterial agent that exhibits an EC₅₀ of 10-15 µM or less, or typically less than 1-5 µM, is desirable.

Generally, the Kit comprises a container and a label or package insert on or associated with the container. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle or an aerosol spray device). The label or package insert indicates that the composition is used for treating the condition of choice, such as bacteria infections.

Alternatively, or additionally, the Kit may further comprise a second (or third) container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The foregoing description will be more fully understood with reference to the following Examples. Such Examples, are, however, exemplary of methods of practicing the present invention and are not intended to limit the scope of the invention.

### EXAMPLES

### Example 1

### Material And Methods

### Bacteria strains.

Different strains of gram-positive bacteria, e.g., *Staphylococcus aureus, Bacillus subtilis, Micrococcus luteus*, and different strains of gram-negative bacteria, e.g., *Escherichia coli, pseudomonas aeruginosa*, were used. Bacterium were grown and maintained according to standard protocols known in the art.

### nPLVs preparation.

The primary lipid used was polyoxyethylene cetyl ether (PCE), either alone or in combination with palmitic acid (PA), hexadecyl trimethylammonium bromide (HTAB) or dimethyldihexadecyl ammonium bromide (DHAB) at the molar ratios set forth below.

The lipid mixture was heated to 50°C and mixed with phosphate buffer saline (PBS), also pre-heated to 50°C, using the 2-syringes method. Briefly, a 10-ml syringe, containing 0.5 g of the lipid mixture, was connected to a second 10-ml syringe containing 10 ml of phosphate buffer saline (PBS) (5 % final lipid concentration). The lipid blend was then injected into the PBS syringe, and the resulting mixture was rapidly passed forth and back 20 times, until a homogeneous suspension was obtained. The preparation was subsequently checked for nPLV quality by phase-contrast microscopy. Four types of nPLVs were prepared: nPLV01: 100% polyoxyethylene cetyl ether (PCE); nPLV02: 99.9% PCE and 0.1% palmitic acid (PA); nPLV03 99.9% PCE and 0.1% hexadecyl trimethylammonium bromide (HTAB); nPLV04 99.9% PCE and 0.1% dimethyldihexadecyl ammonium bromide (DHAB). Two sets of each type of nPLVs were prepared: one with 5 mM cyclodextrin and the other containing no cyclodextrin.

### Inactivation assay.

Following plating on agar of the indicated bacteria, a drop of nPLV compositions, with or without cyclodextrin, was deposited on the plates. For each product a volume of 100 µl was deposited at a concentration of 0.5 mg/ml (50 mg total lipid). Agar plates were then incubated at 37°C overnight. White plaques indicated an inhibition of growth.

### Example 2: Determination of minimum inhibitory concentration (MIC)

### Bacteria strain.

Bacillus subtilis was used in this experiment.

### nPLVs preparation.

As previously described in example 1.

### Assay.

Inoculums of 5x10⁵ bacteria/ml (b. subtilis) were grown overnight in LB broth medium in presence of various concentration of nPLV03 (99.9% PCE and 0.1% hexadecyl trimethylammonium bromide (HTAB)). Each culture sample was then serially diluted and plated on agar for titer determination.

### Example 3 - Results

In order to explore the possibility of inhibiting bacteria using the composition disclosed herein, Applicants tested the inhibitory effect of the composition on both gram-positive and gram-negative bacteria.

Applicants synthesized four different nPLVs using different lipid compounds: nPLV01: 100% polyoxyethylene cetyl ether (PCE); nPLV02: 99.9% PCE and 0.1% palmitic acid (PA); nPLV03 99.9% PCE and 0.1% hexadecyl trimethylammonium bromide (HTAB); nPLV04 99.9% PCE and 0.1% dimethyldihexadecyl ammonium bromide (DHAB). Also, two sets of each type of nPLVs were prepared: one with 5 mM cyclodextrin and the other contains no cyclodextrin.

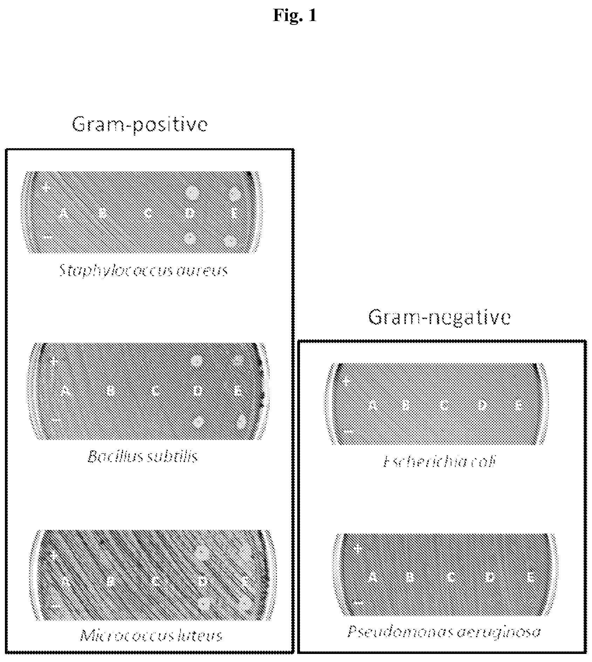

As evident from figure 1, vesicles containing a quaternary ammonium (50 ng total) inhibited the growth of bacteria. This inhibitory effect appeared, at this concentration, to be independent of the presence of cyclodextrin.

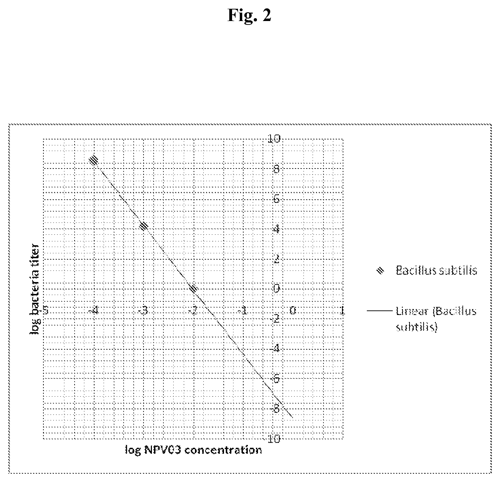

As evident from Figure 2, minimum inhibitory concentration for nPLV03 (without cyclodextrin) was determined as 23.4 mg/L of total lipid, i.e. 23.4 µg/L of HTAB.

### REFERENCES

Adam CD, Durrant, JA, Lowry MR, Tiddy G. J. Gel and liquid-crystal Phase structures of the trioxyethyleneglycol monohexadecyl ether/water system. J. Chem. Soc. Faraday Trans 1984;80:789-01.
Aloia, RC, Tian H, Jensen FC. Lipid composition and fluidity of the human immunodeficiency virus envelope and host cell plasma membranes, Proc. Natl Acad Sci 1993;90:5181-85
Bai, J. and R.E. Pagano, Measurement of spontaneous transfer and transbilayer movement of BODIPY-labeled lipids in lipid vesicles. Biochemistry 1997; 36:8840-48.
Brahmbhatt M. Avian influenza: Economic and social impacts. http;web.worldbank.org
Bright RA, Medina M-J, Xu X, Perez-Oronoz G, Wallis TA, , Davis XM, Povinelli L, Coc NJ, Kimov A. Incidence of adamantine resistance among influenza A (H3N2) viruses isolated worldwide from 1994 to 2005; a cause for concern. 2005. Lancet DOL: 10.1016/50140-6736. Sep. 22. pg 1-7
Campanha MTN, Mamizuka EM, Carmona-Ribeiro AM. Interactions between cationic liposomes and bacteria: the physical chemistry of the bactericidal action. Journal Of Lipid Research, Bethesda, MD, US, vol. 40, 1 January 1999, pages 1495-1500.
El Baraka M, Pecheur EI, Wallach DFH, .Philippot JR. Non-phospholipid fusogenic liposomes. Biochim. Biophys. Acta 1996; 1280, 107-114.
Greenberg M and Cammack N. Resistance to enfurvirtide the first HIV fusion inhibitor. 2004. J. Antimicrobial Chemotherapy 54;:333-40.
Heerklotz H. Triton promotes domain formation in lipid rafts. Biophys J 2002;83: 2693-701.
Hersberger M, Nusbaumer C, Scholer A, Knöpfli V, Eckardstein AV. Influence of practicable virus inactivation procedures on tests for frequently measured analytes in plasma. Clin Chem 2004;50;944-46.
HIV/AIDS and work: global estimates, impact and response 2004; ILO Programme on HIV/AIDS and the world of work.
Jefferson T, Rivetti D, Rivetti A, Rudin M, Pietrantonj CD, Demichelli V. Efficacy and effectiveness of influenza vaccines in elderly people: a systematic review. Lancet DOL 1016/50140-.
Kilby J.M., Enron J.J. Mechanisms of disease. Novel therapies based on mechanisms of HIV-1 cell entry. New Engl J Med, 2003,;48: 2228-38.
Lantzsch G, Binder H, Heerklotz H, Wendling M. Klose W. Surface areas and packing constraints in POPC/C12EOn membranes; a time-resolved fluorescence study. Biophys Chem , 1996; 58: 289-02.
M cLean LR, Phillips M.C. Mechanism of cholesterol and phosphatidylcholine exchange or transfer between unilamellar vesicles. Biochemistry 1981;12: 2893-90.
Mitchell J, Tiddy, GJT, Waring, L, Bostock T, McDonald M.P. Phase behavior of polyoxyethylene surfactants with water. J. Chem. Soc. Faraday Trans 1983, 79:975-1000.
Moscona.A. NANASE Inhiibitors for influenza. New. Engl. J. Med. 2005:353;1363-73..
Mukherjee S, Chattopadhyay A., Membrane organization at low cholesterol concentrations: a study using 7-nitrobenz-2-oxa-1,3-diazol-4-yl -labelled cholesterol. Biochemistry.1996; 35:1311-22.
Ono A,. Freed E.O. Plasma membrane rafts play a critical role in HIV-1 assembly and release. Proc Natl Acad Sci 2001; 98: 13925-30.
Pelet T, Miazza V, Mottet G, Roux L. High throughput screening assay for negative single stranded rna virus polymerase inhibitors. J Virol Methods 2005;128:1-2;:29-36.
Rockstroh JK Mauss S. Clinical perspective of fusion inhibitors for treatment of HIV. 2004. J. Antimicrobial Chemotherapy 54:700-702...
Roberts P. Resistance of vaccinia virus to inactivation by solvent/detergent treatment of blood products. Biologicals 200;28:29-32.
Rousso I, Mixon MB, Chen BK, Kim PS. Palmitoylation of the HIV-1 envelope glycoprotein is critical for viral infectivity. Proc Natl Acad Sci 2000;97:1353-25.
Scheiffele P, Rietveld A, Wilk T, Simons K. Influenza viruses select ordered lipid domains during budding from the plasma membrane, J Biol Chem , 1999; 274:2038-44.
Simons K, Ehehalt R. Cholesterol, lipid rafts and disease, J Clin Invest 2002;110:597-03.
Small DJ. Role of ABC transporters in secretion of cholesterol from liver to bile. Proc Natl Acad Sci 2002; 100:4-7.
Steck TL, Straus JH, Wallach D.F.H. A model for the behavior of vesicles in density gradients: Implications for fractionation. Biochim Biophys. Acta 1970;.23:385-93.
Steck TL, Ye J, Lange Yvonne. Probing red cell membrane cholesterol movement with cyclodextrin; Biophys J 2000,;283:2118-25.
Tashima KT, Carpenter C.C.J. Fusion Inhibition - A major but costly step forward in the treatment of HIV-1. N Engl J Med. 2003; 348:2249-22.
Taubenberger JK, Reid AH, Lourens RM,, Wang R, Jin G, Fanning TG. Characterization of the 1918 influenza virus polymerase genes. 2005. Nature 437/6:889-93.
Tumpey, TT, Basler CF, Aguillar PV, Zeng H,Solorzano A, Swayne DE, Cox NJ, Katz JM, Characterization of the reconstructed 1918 spanish influenza pandemic virus. 2005. Science;310:77-80.
Varanelli C, Kumar S. Wallach D.F.H. 1996, Method of inhibiting viral reproduction using nonphospholipid vesicles. U.S. Patent 5,561,062
Wallach DFH1990a,. Lipid vesicles formed of surfactants and steroids. U.S. Patent 4,197,951.
Wallach DFH. 1990b Paucilamellar lipid vesicles. U.S. Patent 4,911,928.
Wallach DFH. 1992, Paucilamellar lipid vesicles. U.S. Patent 5,147,723.
Wallach DFH. 1996, Paucilamellar lipid vesicles. U.S. Patent 5,474,848
Wallach DFH., 1997, Hybrid paucilamellar lipid vesicles. U.S. Patent 5,628,936.
Wallach DFH, Varanelli C., 1997, Lipid vesicle fusion as a method of transmitting a biologically active material to a cell. U.S .Patent 5,665,380.
Wallach DFH1990a,. Lipid vesicles formed of surfactants and steroids. U.S. Patent 4,197,951.
Wallach DFH. 1990b Paucilamellar lipid vesicles. U.S. Patent, 4,911,928.
Wallach DFH. 1992, Paucilamellar lipid vesicles. U.S. Patent 5,147,723.
Wallach DFH. 1996, Paucilamellar lipid vesicles. U.S. Patent 5,474,848
Wallach DFH., 1997, Hybrid paucilamellar lipid vesicles. U.S. Patent 5,628,936.
Wallach DFH, Varanelli C., 1997, Lipid vesicle fusion as a method of transmitting a biologically active material to a cell. U.S .Patent 5,665,380.
Wallach DFH New non-phospholipid vesicles (nPLV) and their use in cosmetic, therapeutic and prophylactic applications, 2001. European Patent application 1, 304,103, PCT, US extension 2005.
Working committee of the World Health organisation (Who). Avian influenza (H5N1) infection
Wallach DFH New non-phospholipid vesicles (nPLV) and their use in cosmetic, therapeutic and prophylactic applications, 2001. European Patent application 1, 304,103, PCT, US extension 2005.

## Claims

1. A composition comprising at least one non-phospholipid lipid vesicle (nPLV) for use in the treatment, inhibition or prevention of a disorder associated with gram-positive bacteria wherein said nPLV comprises a lipid comprising a quaternary ammonium head group and the molar ratio of said lipid is from 0.01 % to 1%.

2. The composition of claim 1 for use in the treatment, inhibition or prevention of a disorder associated with gram-positive bacteria wherein said lipid is selected from the group consisting of hexadecyl trimethylammonium bromide (HTAB), dimethyldihexadecyl ammonium bromide (DHAB), Cetrimonium chloride, cocamidopropyl betaine (CAPB), and tetra-n-butylammonium bromide (TBAB).

3. The composition of claim 1 for use in the treatment, inhibition or prevention of a disorder associated with gram-positive bacteria wherein the molar ratio of said lipid is from 0.1% to 0.5%.

4. The composition of claim 1 for use in the treatment, inhibition or prevention of a disorder associated with gram-positive bacteria wherein said nPLV is cholesterol free.

5. The composition of claim 1 or claim 2 for use in the treatment, inhibition or prevention of a disorder associated with gram-positive bacteria wherein said nPLV is unilamellar, paucillamelar or multilamellar.

6. The composition of claim 1 wherein said composition comprises a cyclodextrin or derivatives thereof.

7. The composition of claim 6 wherein the cyclodextrin is selected from the group consisting of α-cyclodextrin, β-cyclodextrin, γ-cyclodextrin and a combination thereof.

8. The composition of claim 6 wherein the derivatives are selected from the group consisting of dimethyl- β-cyclodextrin, trimethyl- β-cyclodextrin, randomly methylated- β-cyclodextrin, hydroxyethyl- β-cyclodextrin, 2-hydroxypropyl- β-cyclodextrin, 3-hydroxypropyl- β-cyclodextrin, 2,3-dihydroxypropyl- β-cyclodextrin, 2-hydroxyisobutyl- β-cyclodextrin, sulphobutylether- β-cyclodextrin, glucosyl- β-cyclodextrin, maltosyl- β-cyclodextrin and combinations thereof.

9. The composition of claim 1 for use in the treatment, inhibition or prevention of a disorder associated with gram-positive bacteria, wherein said gram-positive bacteria is selected from the group consisting of Staphylococcus aureus, Staphylococcus epidermidis, Staphylococcus sp. Coagulase-negative, Streptococcus pneumoniae of Viridans group, Streptococcus agalactiae of group B, Streptococcus spyogenes, Enterococcus sp., Bacillus anthracis, Bacillus oereus, Bifidobacteriu bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., and Peptostreptococcus sp.

10. The composition of claim 1 for use in the treatment, inhibition or prevention of a disorder associated with gram-positive bacteria, wherein said composition is administered to a mammal.

11. The composition of claim 1 for use in the treatment, inhibition or prevention of a disorder associated with gram-positive bacteria, wherein said composition is administered to a human.

12. The composition of claim 1 for use in the treatment, inhibition or prevention of a disorder associated with gram-positive bacteria, wherein said disorder is an infection.

13. The composition of claim 12 for use in the treatment, inhibition or prevention of a disorder associated with gram-positive bacteria, wherein said disorder is a drug resistant and/or multiple-drug resistant bacterial infection.

14. The composition of claim 13 for use in the treatment, inhibition or prevention of a disorder associated with gram-positive bacteria, wherein said infection is from one or more of methicillin resistant Staphylococcus aureus (MRSA), methicillin resistant Staphylococcus epidermidis (MRSE), methicillin resistant coagulase negative Staphylococci (MRCNS), vancomycin resistant Enterococcus faecalis, and/or vancomycin resistant Enterococcus faecium.

15. The composition of claim 1 for use in the treatment, inhibition or prevention of a disorder associated with gram-positive bacteria, wherein said nPLV is coadministered with at least one other antibacterial agent.

16. The composition of claim 15 for use in the treatment, inhibition or prevention of a disorder associated with gram-positive bacteria, wherein said at least one other antibacterial agent is selected from the group consisting of aminoglycosides, β-lactam antibiotics, cephalosporius, macrolides, miscellaneous antibiotics, penicillins, tetracyclines, antifungals, antimalarial agents, antituberculosis agents, antibacterials, leprostatics, miscellaneous anti-infectives, quinolones, sulfonamides, urinary anti-infectives, nasal antibiotics, opthalmic antibiotics, opthalmic antibacterials, opthalmicquinalones, opthalmic sulfonamides, skin and mucous membrane antibiotics, skin and mucous membrane antifungals, skin and mucous membrane antibacterials, skin and mucous membrane miscellaneous anti-infectives, skin and mucous membranescabicides and pedulicides, skin and mucous membrane antineoplasts, nitrofurans and oxazolidinones.

17. The composition of claim 15 for use in the treatment, inhibition or prevention of a disorder associated with gram-positive bacteria, wherein said nPLV is administered simultaneously or sequentially with said at least one other antibacterial agent.

18. A method for increasing the antibacterial activity of an object comprising contacting said object with a composition of any one of claims 1-17.

19. The method of claim 18 wherein said object is selected from the group consisting of medical equipment, medical devices, and hospital supplies.

20. The method of claim 18 wherein said object is selected from the group consisting of a surface, surgical gloves, surgical instruments and personal masks.

21. The method of claim 18 wherein said composition is in the form of an aqueous suspension or dispersion in hydrogels.

22. A kit suitable for treating, inhibiting or preventing an infection of gram-positive bacteria in a subject, said kit comprising a composition consisting of non-phospholipid lipid vesicles (nPLV) wherein said nPLV consist of polyoxyethylene cetyl ether and a lipid comprising a quaternary ammonium head group and the molar ratio of said lipid is from 0.01% to 1%, in combination with reagents and/or instructions for use.

## Patentansprüche

1. Stoffgemisch enthaltend mindestens eine nichtphospholipiden Lipidvesikel (nPLV) zur Anwendung bei der Behandlung, Hemmung oder Vorbeugung von einer mit grampositive Bakterien verbundenen Krankheit, worin das besagte nPLV ein Lipid mit einer quaternären Ammonium-kopfgruppe umfasst, und das Molverhältnis des besagten Lipids von 0.01% bis 1% beträgt.

2. Stoffgemisch nach Anspruch 1 zur Anwendung bei der Behandlung, Hemmung oder Vorbeugung von einer mit grampositive Bakterien verbundenen Krankheit, worin das besagte Lipid ausgewählt wird aus der Gruppe bestehend aus: Hexadecyl Trimethylammonium-Bromid (HTAB), Dimethyldihexadecyl Ammoniumbromid (DHAB), Cetrimonium-Chlorid, Cocamidopropyl-Betain (CAPB) und Tetra-n-Butylammonium-Bromid (TBAB).

3. Stoffgemisch nach Anspruch 1 zur Anwendung bei der Behandlung, Hemmung oder Vorbeugung von einer mit grampositive Bakterien verbundenen Krankheit, worin das Molverhältnis des besagten Lipids von 0.1% bis 0.5% beträgt.

4. Stoffgemisch nach Anspruch 1 zur Anwendung bei der Behandlung, Hemmung oder Vorbeugung von einer mit grampositive Bakterien verbundenen Krankheit, worin das nPLV cholesterinfrei ist.

5. Stoffgemisch nach Anspruch 1 oder Anspruch 2 zur Anwendung bei der Behandlung, Hemmung oder Vorbeugung von einer mit grampositive Bakterien verbundenen Krankheit, worin das besagte nPLV unilamellar, paucillamellar oder multilamellar ist.

6. Stoffgemisch nach Anspruch 1, worin die besagte Stoffgemisch ein Cyclodextrin oder Derivate davon umfasst.

7. Stoffgemisch nach Anspruch 6, worin das Cyclodextrin ausgewählt wird aus der Gruppe bestehend aus: α-Cyclodextrin, β-Cyclodextrin, γ-Cyclodextrin und einer Kombination davon.

8. Stoffgemisch nach Anspruch 6, worin die Derivate ausgewählt werden aus der Gruppe bestehend aus: Dimethyl-β-Cyclodextrin, Trimethyl-β-Cyclodextrin, zufällig methyliertes β-Cyclodextrin, Hydroxyaethyl-β-Cyclodextrin, 2-Hydroxypropyl-β-Cyclodextrin, 3-Hydroxypropyl-β-Cyclodextrin, 2.3-Dihydroxyporpyl-β-Cyclodextrin, 2-Hydroxyisobutyl-β-Cyclodextrin, Sulphobutylether-β-Cyclodextrin, Glucosyl-β-Cyclodextrin, Maltosyl-β-Cyclodextrin und Kombinationen davon.

9. Stoffgemisch nach Anspruch 1 zur Anwendung bei der Behandlung, Hemmung oder Vorbeugung von einer mit grampositive Bakterien verbundenen Krankheit, worin das besagte grampositiv Bakterium ausgewählt wird aus der Gruppe bestehend aus: *Staphylococcus aureus, Staphylococcus epidermidis*, koagulasenegativem *Staphylococcus sp.*, *Streptococcus pneumoniae* aus der Gruppe *Viridans, Streptococcus agalactiae* der Gruppe B, *Streptococcus spyogenes, Enterococcus sp.*, *Bacillus anthracis, Bacillus oereus*, *Bifidobacterium bifidum, Lactobacillus sp.*, *Listeria monocytogenes*, *Nocardia sp.*, *Rhodococcus equi*, *Erysipelothrix rhusiopathiae, Corynebacterium diptheriae, Propionibacterium acnes, Actinorrayces sp., Clostridium botulinum, Clostridium difficile, Clostridium perfringens, Clostridium tetani, Mobiluncus sp.*, und *Peptostreptococcus sp.*

10. Stoffgemisch nach Anspruch 1 zur Anwendung bei der Behandlung, Hemmung oder Vorbeugung von einer mit grampositive Bakterien verbundenen Krankheit, worin die besagte Stoffgemisch einem Säugetier verabreicht wird.

11. Stoffgemisch nach Anspruch 1 zur Anwendung bei der Behandlung, Hemmung oder Vorbeugung von einer mit grampositive Bakterien verbundenen Krankheit, worin die besagte Stoffgemisch einem Menschen verabreicht wird.

12. Stoffgemisch nach Anspruch 1 zur Anwendung bei der Behandlung, Hemmung oder Vorbeugung von einer mit grampositive Bakterien verbundenen Krankheit, worin die besagte Störung eine Infektion ist.

13. Stoffgemisch nach Anspruch 12 zur Anwendung bei der Behandlung, Hemmung oder Vorbeugung von einer mit grampositive Bakterien verbundenen Krankheit, worin die besagte Störung eine arzneimittelresistente und/oder multiarzneimittelresistente bakterielle Infektion ist.

14. Stoffgemisch nach Anspruch 13 zur Anwendung bei der Behandlung, Hemmung oder Vorbeugung von einer mit grampositive Bakterien verbundenen Krankheit, worin die besagte Störung methicillinresistentes *Staphylococcus aureus* (MRSA), methicillinresistentes *Staphylococcus epidermidis* (MRSE), methicillinresistente coagulasenegative *Staphylococci* (MRCNS), Vancomycin-resistentes *Enterococcus faecalis* und/oder Vancomycin-resistentes *Enterococcus faecium* ist.

15. Stoffgemisch nach Anspruch 1 zur Anwendung bei der Behandlung, Hemmung oder Vorbeugung von einer mit grampositive Bakterien verbundenen Krankheit, worin das besagte nPLV zusammen mit mindestens einem anderen antibakteriellen Mittel verabreicht wird.

16. Stoffgemisch nach Anspruch 15 zur Anwendung bei der Behandlung, Hemmung oder Vorbeugung von einer mit grampositive Bakterien verbundenen Krankheit, worin das andere antibakterielle Mittel ausgewählt wird aus der Gruppe bestehend aus: Aminoglycosid, Beta-Lactam-Antibiotika, Cephalosporinen, Makroliden, verschiedenen Antibiotika, Penizillinen, Tetracyclinen, Antimykotika, Antimalariamittel, Antituberkulotika, Antibakterika, Leprostatika, verschiedenen Anti-Infektiva, Quinolonen, Sulfonamiden, Harnweg-Anti-Infektiva, Nasenantibiotika, Augenantibiotika, Augenantibakterika, Augenquinolonen, Augensulfonamiden, Antibiotika für Haut und Schleimhaut, Antimykotika für Haut und Schleimhaut, Antibakterika für Haut und Schleimhaut, verschiedenen Anti-Infektiva für Haut und Schleimhaut, Krätzeheilmitteln und Entlausungsmitteln für Haut und Schleimhaut, Antineoplasten für Haut und Schleimhaut, Nitrofuranen und Oxazolidinonen.

17. Stoffgemisch nach Anspruch 15 zur Anwendung bei der Behandlung, Hemmung oder Vorbeugung von einer mit grampositive Bakterien verbundenen Krankheit, worin das besagte nPLV gleichzeitig oder sequentiell mit dem besagten mindestens einen anderen antibakteriellen Mittel verabreicht wird.

18. Verfahren zur Erhöhung der antibakterielle Tätigkeit eines Objektes, umfassend das Kontaktieren des besagten Objektes mit einer Stoffgemisch von irgendeinem der Ansprüche 1 bis 17.

19. Verfahren nach Anspruch 18, worin das besagte Objekt ausgewählt wird aus der Gruppe bestehend aus: medizinischer Ausrüstung, medizinischen Apparaten und Krankenhauszubehör.

20. Verfahren nach Anspruch 18, worin das besagte Objekt ausgewählt wird aus der Gruppe bestehend aus: einer Oberfläche, chirurgischen Handschuhen, chirurgischen Instrumenten und persönlichen Schutzmasken.

21. Verfahren nach Anspruch 18, worin die besagte Stoffgemisch in der Form einer wässrigen Suspension oder einer Hydrogeldispersion vorliegt.

22. Kit, geeignet zur Verwendung in der Behandlung, Hemmung oder Vorbeugung einer mit grampositive Bakterien verbundenen Krankheit bei einem Subjekt, wobei das besagte Kit eine Stoffgemisch bestehend aus nichtphospholipiden Lipidvesikeln (nPLV) umfasst, worin das besagte nPLV aus Polyoxyethylene-Cetyl-Ether und aus einem Lipid mit einer quaternären Ammoniumkopfgruppe besteht, und das Molverhältnis des besagten Lipids von 0.01% bis 1% beträgt, in Kombination mit Reagenzien und/oder Gebrauchsanweisungen.

## Revendications

1. Une composition comprenant au moins une vésicule lipidique non-phospholipidique (nPLV) destinée à être utilisée dans le traitement, l'inhibition ou la prévention d'une maladie associée à des bactéries gram positif, dans laquelle ladite nPLV comprend un lipide comprenant un groupe ammonium quaternaire et le rapport molaire dudit lipide est de 0.01% à 1%.

2. La composition selon la revendication 1, destinée à être utilisée dans le traitement, l'inhibition ou la prévention d'une maladie associée à des bactéries gram positif, dans laquelle ledit lipide est sélectionné parmi le groupe consistant en bromure d'hexadécyle triméthylammonium (HTAB), bromure de diméthyldihexadécyle ammonium (DHAB), chlorure de cétrimonium, cocamidopropyl bétaine (CAPB) et bromure de tétra-n-butylammonium (TBAB).

3. La composition selon la revendication 1, destinée à être utilisée dans le traitement, l'inhibition ou la prévention d'une maladie associée à des bactéries gram positif, dans laquelle le rapport molaire dudit lipide est de 0.1% à 0.5%.

4. La composition selon la revendication 1, destinée à être utilisée dans le traitement, l'inhibition ou la prévention d'une maladie associée à des bactéries gram positif, dans laquelle nPLV est sans cholestérol.

5. La composition selon la revendication 1 ou la revendication 2, destinée à être utilisée dans le traitement, l'inhibition ou la prévention d'une maladie associée à des bactéries gram positif, dans laquelle ladite nPLV est unilamellaire, paucillamellaire ou multilamellaire.

6. La composition selon la revendication 1, dans laquelle ladite composition comprend une cyclodextrine ou leurs dérivés.

7. La composition selon la revendication 6, dans laquelle la cyclodextrine est sélectionnée dans le groupe consistant en α-cyclodextrine, β-cyclodextrine, γ-cyclodextrine et une combinaison de celles-ci.

8. La composition selon la revendication 6, dans laquelle les dérivés sont sélectionnés dans le groupe consistant en diméthyle-β-cyclodextrine, triméthyle-β-cyclodextrine, β-cyclodextrine méthylé aléatoirement, hydroxyéthyle-β-cyclodextrine, 2-hydroxypropyle-β-cyclodextrine, 3-hydroxypropyle-β-cyclodextrine, 2,3-dihydroxypropyle-β-cyclodextrine, 2-hydroxyisobutyle-β-cyclodextrine, sulphobutylether-β-cyclodextrine, glucosyle-β-cyclodextrine, maltosyl-β-cyclodextrine et leurs combinaisons.

9. La composition selon la revendication 1, destinée à être utilisée dans le traitement, l'inhibition ou la prévention d'une maladie associée à des bactéries gram positif, dans laquelle ladite bactérie gram positive est sélectionnée parmi le groupe consistant en Staphylococcus aureus, Staphylococcus epidermidis, Staphylococcus sp. à coagulase négative, Streptococcus pneumoniae du groupe Viridans, Streptococcus agalactiae du groupe B, Streptococcus spyogenes, Enterococcus sp., Bacillus anthracis, Bacillus oereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium *acnes,* Actinomyces sp., Clostridium botulinum, Clostridium difficile, Clostridium perfringens, Clostridium tetani, Mobiluncus sp., et Peptostreptococcus sp.

10. La composition selon la revendication 1, destinée à être utilisée dans le traitement, l'inhibition ou la prévention d'une maladie associée à des bactéries gram positif, dans laquelle ladite composition est administrée à un mammifère.

11. La composition selon la revendication 1, destinée à être utilisée dans le traitement, l'inhibition ou la prévention d'une maladie associée à des bactéries gram positif, dans laquelle ladite composition est administrée à un être humain.

12. La composition selon la revendication 1, destinée à être utilisée dans le traitement, l'inhibition ou la prévention d'une maladie associée à des bactéries gram positif, dans laquelle ledit trouble est une infection.

13. La composition selon la revendication 12, destinée à être utilisée dans le traitement, l'inhibition ou la prévention d'une maladie associée à des bactéries gram positif, dans laquelle ledit trouble est une infection bactérienne résistante aux médicaments et/ou multi-résistante aux médicaments.

14. La composition selon la revendication 13, destinée à être utilisée dans le traitement, l'inhibition ou la prévention d'une maladie associée à des bactéries gram positif, dans laquelle ladite infection est liée à un ou plus d'un Staphylococcus aureus résistant à la méticilline (SARM), Staphylococcus epidermidis résistant à la méticilline (SERM), Staphylocoques à coagulase négative résistants à la méticilline (SCNRM), Enterococcus faecalis résistant à la vancomycine et/ou Enterococcus faecium résistant à la vancomycine.

15. La composition selon la revendication 1, destinée à être utilisée dans le traitement, l'inhibition ou la prévention d'une maladie associée à des bactéries gram positif, dans laquelle ladite nPLV est co-administrée avec au moins un autre agent antibactérien.

16. La composition selon la revendication 15, destinée à être utilisée dans le traitement, l'inhibition ou la prévention d'une maladie associée à des bactéries gram positif, dans laquelle ledit au moins autre agent antibactérien est sélectionné parmi le groupe consistant en aminoglycosides, antibiotiques bêta-lactamines, céphalosporines, macrolides, divers antibiotiques, pénicillines, tétracyclines, antifongiques, agents antipaludiques, agents antituberculeux, antibactériens, léprostatiques, divers anti-infectieux, quinolones, sulfamides, agents anti-infections urinaires, antibiotiques nasaux, antibiotiques ophtalmiques, antibactériens ophtalmiques, quinolones ophtalmiques, sulfamides ophtalmiques, antibiotiques pour la peau et les membranes des muqueuses, antifongiques pour la peau et les membranes des muqueuses, antibactériens pour la peau et les membranes des muqueuses, divers anti-infectieux pour la peau et les membranes des muqueuses, antiscabieux et pédiculicides pour la peau et les membranes des muqueuses, antinéoplasiques pour la peau et les membranes des muqueuses, nitrofuranes et oxazolidinones.

17. La composition selon la revendication 15, destinée à être utilisée dans le traitement, l'inhibition ou la prévention d'une maladie associée à des bactéries gram positif, dans laquelle ladite nPLV est administrée simultanément ou séquentiellement avec ledit au moins autre agent antibactérien.

18. Un procédé pour augmenter l'activité antibactérienne d'un objet comprenant la mise en contact de l'objet avec une composition selon l'une quelconque des revendications 1 à 17.

19. Le procédé selon la revendication 18, dans lequel ledit objet est sélectionné parmi le groupe consistant en équipement médical, dispositifs médicaux et fournitures d'hôpital.

20. Le procédé selon la revendication 18, dans lequel ledit objet est sélectionné parmi le groupe consistant en une surface, des gants chirurgicaux, des instruments chirurgicaux et des masques personnels.

21. Le procédé selon la revendication 18, dans lequel ladite composition est sous la forme d'une suspension aqueuse ou d'une dispersion en hydrogels.

22. Un kit utilisable pour le traitement, l'inhibition ou la prévention d'une maladie associée à des bactéries gram positif dans un sujet, ledit kit comprenant une composition consistant en des vésicules lipidiques non-phospholipidiques (nPLV), dans laquelle ladite nPLV est constituée de polyoxyéthylène cétyle éther et d'un lipide comprenant un groupe ammonium quaternaire et le rapport molaire dudit lipide est de 0.01% à 1%, en combinaison avec des réactifs et/ou des instructions d'utilisation.
